# EUROPEAN PATENT APPLICATION

(11) **EP 3 671 756 A1**
(43) Date of publication of application: **24.06.2020**
(21) Application number: 18215490.6
(22) Date of filing: 21.12.2018
(51) Int. Cl.: G16H 30/40, A61C 7/00, A61C 7/08

(54) **COMPUTER-IMPLEMENTED METHOD FOR CREATING A 3D-PRINTING FILE OF AT LEAST ONE DENTAL ALIGNER FOR DENTAL SELF-TREATMENT OF A CUSTOMER BASED ON AT LEAST ONE DIGITAL IMAGE OF THE CUSTOMER'S TEETH, AND DATA PROCESSING SYSTEM FOR CARRYING OUT THE STEPS OF THE METHOD**

(71) Applicant: Lineacura GmbH, 21521 Aumühle (b. Hamburg) (DE)
(72) Inventor: PALTI, Ady, 76530 Baden-Baden (DE); HAGEDORN, Martin, 17893 Drottningholm (SE); BOWIEN, Margarete, 21521 Aumühle (DE)
(74) Representative: Grünecker Patent- und Rechtsanwälte PartG mbB

(57) **Abstract**

The present invention relates to a computer-implemented method for creating a 3D-printing file of at least one dental aligner for dental self-treatment of a customer based on at least one digital image of the customer's teeth, and to a data processing system for carrying out the steps of the method. In order to improve the efficiency and precision of the production of customer-adapted dental aligners with a minimum of resources and interaction of dentists with customers and collaboration with dental laboratories, the present invention provides a computer-implemented method for creating at least one 3D-printing file of at least one dental aligner for dental self-treatment of a customer based on at least one digital image of the customer's teeth, wherein the method includes the following steps:
Step A: Verifying whether or not the at least one digital image satisfies preset requirements on image eligibility and/or customer eligibility.
Step B: If the verification in Step A is positive, creating the at least one 3D-printing file for 3D-printing the at least one dental aligner based on the at least one digital image.

A data processing system for carrying out the steps of the method is also disclosed.

## Description

The present invention relates to a computer-implemented method for creating a 3D-printing file of at least one dental aligner for dental self-treatment of a customer based on at least one digital image of the customer's teeth, and to a data processing system for carrying out the steps of the method.

A dental service is known which improves teeth alignment to its customers via minor teeth correction(s) using dental aligners.

Dental aligners are a proven successful dental device for many years and used by millions of customers/patients. However, the preparation of customer-adapted dental aligners requires resources through personal and physical interaction of dentists with customers and collaboration with dental laboratories.

The objective of the present invention is to improve the efficiency and precision of the production of customer-adapted dental aligners with a minimum of resources and interaction of dentists with customers and collaboration with dental laboratories.

The objective is solved by the computer-implemented method according to claim 1 and the data processing system according to claim 5.

The computer-implemented method according to claim 1 is a method for creating at least one 3D-printing file of at least one dental aligner for dental self-treatment of a customer based on at least one digital image of the customer's teeth, wherein the method includes the following steps:
- Step A: Verifying whether or not the at least one digital image satisfies preset requirements on image eligibility and/or customer eligibility.
- Step B: If the verification in Step A is positive, creating the at least one 3D-printing file for 3D-printing the at least one dental aligner based on the at least one digital image.

The claimed invention is driven by the idea of minimizing or entirely dispensing with the use of resources and interaction of dentists with customers and collaboration with dental laboratories, and to have essential steps accomplished by the customer itself. In particular, the customer who is interested in dental self-treatment is to contribute to the treatment by taking and providing at least one digital image of his teeth for the preparation of at least one customer-adapted dental aligner, wherein the claimed method supports the customer in accomplishing these tasks. The number of digital images and dental aligners used in context with this method is not limited to one. The term "at least one" is meant to encompass any integer, preferably from 1 to 100 and even more preferably from 1 to 12. It is particularly envisaged that the steps A and B of the method are repeatedly carried out and that different 3D-printing files for 3D-printing different dental aligners are created on the basis of one digital image or different digital images of the customer's teeth. The dental self-treatment preferably continues over a several weeks or several months time, wherein each dental aligner provides minute teeth correction from an initial state when the treatment starts towards a target state when the treatment is accomplished.

The computer-implemented method according to the present invention may be implemented by a web-based application software and/or application software to be installed on a private or public computer. The computer may be a desktop PC, tablet PC or smartphone and the like, and is not limited to a specific embodiment.

The starting point for the claimed method may be an action taken by the customer by opening the application software.

When starting the application software for the first time, the customer will be invited to open a personal account to which the digital images provided by the customer will be allotted, and the customer will be invited to provide contact, shipping and payment details.

After registration, the process continues with the taking and uploading of the digital images and the subsequent image processing.

In this context, the customer may use his own hardware resources for taking and providing digital images, e.g. computers such as desktop PCs, tablet PCs or smartphones, cameras and the like, or may use suitable stationary or mobile equipment offered by a service provider. More specifically, the customer may use his own smartphone for taking the digital image and uploading the same via the Internet onto a server computer for further processing or have the digital image processed by the application software installed on his own device. Irrespective of where the image processing takes place, the claimed method performs eligibility assessment(s) on the digital image(s) of the customer's teeth and, if the image eligibility and customer eligibility assessment(s) come(s) out clear, finally outputs a digital 3D-printing file that may be used for subsequent 3D-printing the customer-adapted dental aligner.

If the customer has no own equipment or does not want to use it, the service provider may offer stationary or mobile equipment such as a service terminal in a public place, e.g. a train station or supermarket, where the customer may take suitable digital images of his teeth. Alternatively, the service provider may ship suitable mobile equipment ("Starter kit", "3D-imaging kit") for taking and providing the digital images to the customer upon demand, preferably in an automated procedure.

Summarizing, based on the digital image(s) of the customer's teeth, the claimed invention provides at least one 3D-printing file for 3D-printing at least one dental aligner to be used for dental self-treatment. The claimed method involves dentists' expertise in context with image processing where image eligibility and/or customer eligibility for the intended dental self-treatment is assessed on the basis of the at least one digital image of the customer's teeth. More specifically, the customer's eligibility for dental self-treatment using dental aligners is preferably assessed on the basis of the size, shape, position and orientation of the customer's teeth in an initial pre-treatment state, which can be extracted from at least one pre-treatment digital image of the customer's teeth. The step of assessing the customer's eligibility is preferably only performed if both image quality and image content are determined to be sufficient for performing such customer eligibility assessment. These requirements are checked in Step A of the claimed method. The 3D-printing file will only be created if the checks in Step A of the claimed method come out clear. These assessments are intended to secure that system resources will be efficiently used.

The 3D-printing of the dental aligner is not necessarily part of the claimed method as it can be accomplished by other natural or legal persons than the service provider who offers the creation of the 3D-printing file according to the claimed method.

The method according to claim 1 is entirely computer-implemented. Hence, no additional person has to be involved in the entire process apart from the customer itself. Therefore, the method according to the claimed invention provides the technical effect of improving efficiency and precision of the hitherto known production processes of providing customer-adapted dental aligners while requiring a minimum of resources and interaction of dentists with customers and collaboration with dental laboratories.

Preferred embodiments are claimed in the subclaims.

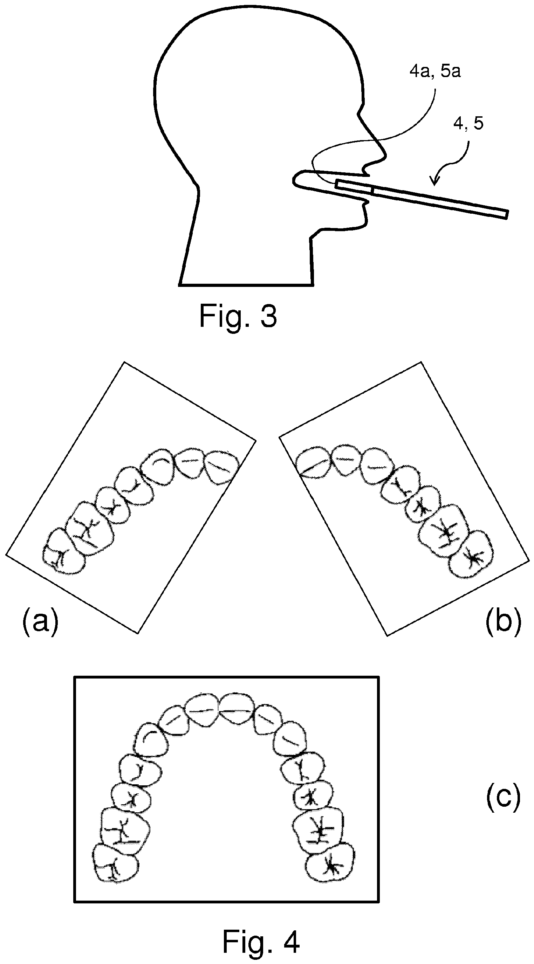

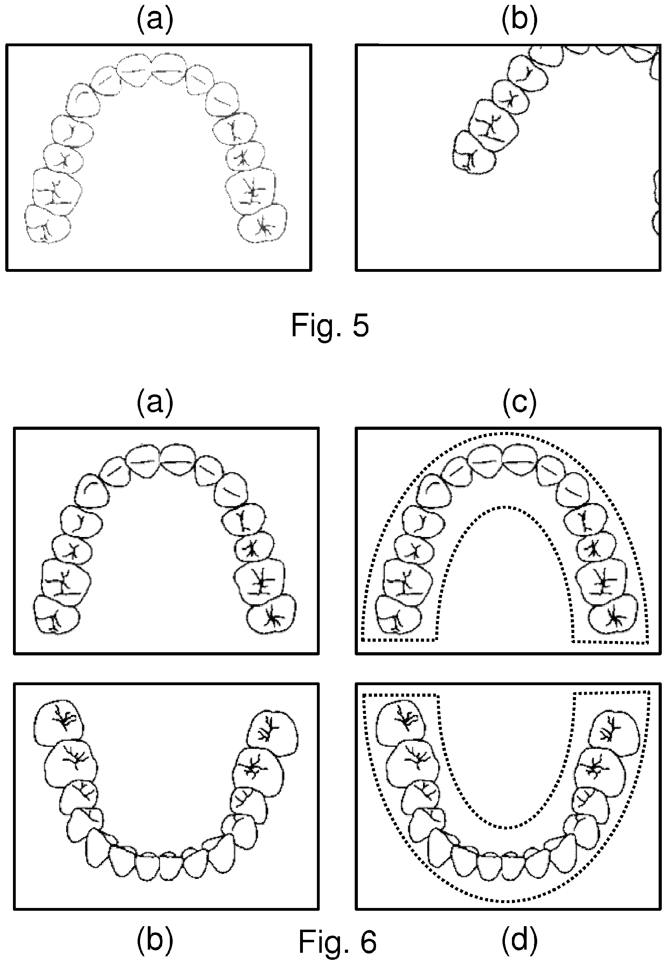

It can prove beneficial if Step A comprises at least one of the following Sub-Steps:
- Sub-Step A1: Triggering shipment of a starter kit to the customer for assisting the customer in taking the at least one digital image. This step may assist the customer in taking the at least one digital image in the most efficient way so as to streamline the subsequent method steps. If the digital image is taken with a view to satisfying the image quality and image content requirements to be subsequently tested, reiterating the initial step of taking the digital images can be avoided.
- Sub-step A2: Loading the at least one digital image, preferably from a data storage device onto which the at least one digital image has been uploaded. The invention is driven by the idea to have most of the required human work done by the customer. In this context, it proves beneficial that the customer prepares the digital image of its teeth itself and uploads these images onto a database of e.g. a server computer for subsequent processing. If sufficient system resources are available, the at least one digital image can be stored or processed with the application software installed on the customer's private device without the need for transferring the digital image onto a server computer via the Internet.
- Sub-step A3: Combining at least two different of said digital images to form a combined digital image of the customer's teeth, wherein the combined digital image preferably depicts at least some or all teeth of the upper jaws and/or lower jaws of the customer, wherein more preferably said combined digital image replaces the at least one digital image in at least one of the subsequent method steps. This Sub-Step may simplify the subsequent processing work. With modern image processing software, adjacent or overlapping images can combined e.g. by using object recognition technologies. If e.g. a first digital image (Fig. 4a) depicts a first subset of the customer's teeth and a second digital image (Fig. 4b) depicts a second subset of the remaining customer's teeth of the customer's upper and/or lower jaws, both digital images may be combined so that the combined image (Fig. 4c) depicts the whole set of customer's teeth of the customer's upper and/or lower jaws. Such image combination can be accomplished with even more than two pictures. This Sub-Step avoids the need for taking a single digital image that depicts the whole set of customer's teeth of the customer's upper and/or lower jaws as such. It also allows minimizing the image size of digital images to be transferred via the Internet. Moreover, this Sub-Step significantly simplifies meeting the image content requirements to be satisfied in context with the subsequent Sub-Step A4.
- Sub-Step A4: Verifying whether or not the at least one digital image satisfies preset image eligibility requirements on image quality and/or image content, preferably including at least one of the following Sub-Steps:
   ∘ Sub-Step A4-1: Setting a parameter requirement for at least one image eligibility parameter representative of eligible image quality and/or eligible image content, said at least one image eligibility parameter preferably including the format and/or size and/or resolution and/or brightness and/or contrast and/or sharpness of the at least one digital image, and/or the number and/or type of teeth depicted in at least one digital image, said image eligibility parameter requirement being more preferably editable and/or derived from a catalogue of eligible images. This Sub-Step prevents that image processing is being carried out on digital images with insufficient quality or content, so that the system resources can be efficiently used. In general, it can be noted that deficiencies in image quality generally occur due to inappropriate imaging settings regarding the above identified parameters format and/or size and/or resolution and/or brightness and/or contrast and/or sharpness. In consequence, modification of imaging settings so as to improve the insufficient image eligibility parameter may help to overcome the verification in the subsequent Sub-Step A4-3. The parameter requirements for the image eligibility parameter are to be set such that the required information on the customer's eligibility for dental self-treatment can be most efficiently extracted from the digital image. A catalog of eligible images may provide guidance for the correct parameter requirement setting.
   ∘ Sub-Step A4-2: Parameterizing the at least one digital image so as to determine at least one parameter value from the at least one digital image for enabling comparison with the parameter requirement set in Sub-Step A4-1. This Sub-Step targets to quantify the image quality in terms of parameter values. As an example, the image resolution can be quantified in terms of a number of pixels contained in the image. Brightness, contrast and sharpness of the image can also be quantified by other parameter values known in the field of (digital) imaging.
   ∘ Sub-Step A4-3: Verifying whether or not the at least one parameter value determined in Sub-Step A4-2 complies with the parameter requirement set in Sub-Step A4-1. This sub-step compares the parameter values attributed to the digital image with the image eligibility parameter(s) preset in advance. This Sub-Step allows efficient automated assessment of whether or not the digital image is eligible for further processing in terms of quality as well as content. In consequence, only those digital images with sufficient quality and content may be rendered subject to the subsequent assessment of customer eligibility for dental self-treatment. As such, system resources can be efficiently used.
   ∘ Sub-Step A4-4: If the verification in Sub-Step A4-3 is negative, rendering a message to the customer, inviting the customer to provide at least another digital image in order to satisfy the preset image eligibility requirement(s), and/or repeating Sub-Step A2 and/or Sub-Step A3 on the basis of the at least another digital image. This Sub-Step applies in case the digital image does not satisfy the preset image eligibility requirements, i.e. the quality of the digital image has been determined to be insufficient for the subsequent customer eligibility assessment. In order to avoid the termination of the entire process at this point in time, the customer is given another possibility to provide a digital image of better quality that might be sufficient for the subsequent customer eligibility assessment. In an example, a digital image is subjected to the image eligibility assessment performed by the application software, wherein the application software outputs information to the customer that the digital image does not meet the preset image eligibility requirements and requests the customer to provide another digital image of the customer's teeth of better quality for the subsequent customer eligibility assessment.
   ∘ Sub-Step A4-5: If the verification in Step A4-3 is positive, proceeding with the method, preferably with Sub-Step A5. If the digital image of the customer's teeth has passed the image eligibility assessment, the process may finally advance to the customer eligibility assessment. As such, only digital images of sufficient quality will be rendered subject to the customer eligibility assessment, so that available system resources will be most efficiently used.
- Sub-Step A5: Verifying whether or not the at least one digital image satisfies preset customer eligibility requirements, preferably including at least one of the following Sub-Steps:
   ∘ Sub-Step A5-1: Setting a parameter requirement for at least one customer eligibility parameter representative of an eligible customer for dental self-treatment with the at least one dental aligner, said at least one customer eligibility parameter preferably including the shape and/or size and/or position and/or orientation of at least one tooth of the customer, more preferably the relative shape and/or size and/or position and/or orientation of at least at least two teeth of an eligible customer, wherein the at least two teeth are even more preferably two adjacent teeth or all (present) teeth of the upper jaws and/or lower jaws of an eligible customer, said customer eligibility parameter requirement being even more preferably editable and/or derived from a catalogue of images of eligible customers. The customer eligibility assessment is an assessment that determines whether or not the customer is eligible for the dental self-treatment in view of the physical condition of its teeth. As an example, if some of the customer's teeth are overlapping or excessively offset such that dental self-treatment with dental aligners is impossible, the customer eligibility assessment would turn out negative. The same applies if the customer has extraordinary teeth defects, such as fractures, malignant growth or chippings which disallow dental self-treatment. Such shape defects may be found out from the digital image by object recognition technology. Setting a parameter requirement for at least one customer eligibility parameter such as shape and/or size and/or position and/or orientation of the customer's teeth (as schematically indicated by the dotted lines in Figs. 6c and 6d) allows quantifying the required minimum physical condition of teeth for the dental self-treatment using the dental aligners to be prepared on the basis of the digital image, and comparison with the quantified physical condition of the customer's teeth. Setting of customer eligibility parameters should be made by involving dentists' expertise.
   ∘ Sub-Step A5-2: Parameterizing the at least one digital image so as to determine at least one parameter value from the at least one digital image for enabling comparison with the parameter requirement set in Sub-Step A5-1, preferably using object recognition technology. This Sub-Step quantifies the physical condition of customer's teeth on the basis of the digital image with a view to the subsequent customer eligibility assessment. As an example, the customer's teeth depicted in the digital images of Figs. 6a to 6d can be parameterized by identifying the coordinates of the outlines of the teeth depicted in the respective digital image(s) as parameter values. This can be accomplished by edge detection technology which is well-known in the field of image processing. These teeth coordinate values correspond to the parameter values referred to in Sub-Step A5-2. The parameter requirement referred to in Sub-Step A5-1 corresponds to the dotted lines in Figs. 6c and 6d.
   ∘ Sub-Step A5-3: Verifying whether or the at least one parameter value determined in Sub-Step A5-2 complies with the parameter requirement set in Sub-Step A5-1. This Sub-Step compares the quantified physical condition of customer's teeth with the minimum physical condition of customer's teeth that is required for the dental self-treatment using the dental aligners. Teeth defects or excessive misalignment of the customer's teeth disabling sample self-treatment with the dental aligners would translate into parameter values exceeding the customer eligibility parameter requirement settings. As such, the Sub-Step A5-3 differentiates between eligible and ineligible customers with respect to the intended dental self-treatment with dental aligners in an automated procedure. In general, on average only about 50% of all people are eligible for dental self-treatment with dental aligners due to the physical condition of their teeth. In the example described with reference to Figs. 6a to 6d, as all of the customer's teeth are within the dotted line, as depicted in Figs. 6c and 6d, the customer is determined to be eligible as the parameter values attributed to the customer's teeth in Sub-Step A5-2 comply with the parameter requirement set in Sub-Step A5-1. On the other hand, if at least one of the customer's teeth would exceed the dotted line, e.g. due to its size shape or relative position or orientation with respect to the other teeth, the customer would be determined to be ineligible as the parameter values attributed to the customer's teeth in Sub-Step A5-2 would not comply with the parameter requirement set in Sub-Step A5-1.
   ∘ Sub-Step A5-4: If the verification in Step A4 is negative, rendering a message to the customer, informing the customer on its ineligibility for the dental self-treatment with dental aligners, and/or referring the customer to a dentist. This Sub-Step terminates the claimed method for all customers which are determined to be ineligible for the dental self-treatment with dental aligners before any cost sensitive actions have been taken. In particular, up to this point in time, no hardware resources such as 3D-printing material and only little image processing resources have been consumed for ineligible customers.
   ∘ Sub-Step A5-5: If the verification in Sub-Step A5-4 is positive, proceeding with the method, preferably with Sub-Step A6, more preferably by rendering a message to the customer informing the customer on its eligibility for the dental self-treatment with dental aligners and/or running a routine for performing a payment procedure and/or running a routine for triggering shipment of a 3D-imaging kit to the customer. This Sub-Step renders the dental self-treatment only available to eligible customers. As cost sensitive actions will be triggered from this point onwards, questions related to payment and debiting arise. And, payment information may be requested from the customer at least at this point in time.
- Sub-Step A6: Simulating the effects of the dental self-treatment of the customer, preferably based on the at least one digital image of the customer's teeth, more preferably including at least one of the following Sub-Steps:
   ∘ Sub-Step A6-1: Computing a target position and/or orientation of at least two teeth or of all teeth of the upper jaws and/or lower jaws of the customer representing a target state upon completion of dental self-treatment with the at least one aligner, preferably based on the at least one digital image. In order to encourage the customer to opt for the dental treatment, it might prove beneficial to tease the customer with an advance presentation of possible results of the dental self-treatment. In this context, while is generally possible to show anonymous standard samples to the customer, it is preferred to present a personalized visualization to the customer, as an anonymous standard sample might be unrealistic in view of the specific physical condition of the respective customer's teeth. In order to provide a more realistic outlook on the expected results of the dental self-treatment, the effect of the dental self-treatment may be simulated on the basis of the digital image that reflects the current physical condition of the customer's teeth. Based on the knowledge of the current physical condition of the customer's teeth, the effects of the dental self-treatment with dental aligners are predictable insofar as intermediate and/or final positions and orientations of the customer's teeth may be computed. Hence, the Sub-Step is regarded as an important aspect for advertising and promoting the services offered by the claimed method but also for the design of the dental aligners to be used in context with the dental self-treatment in order to accomplish teeth alignment in accordance with the computed target position and/or orientation of the customer's teeth. The target state may be a state in which the relative position and/or relative orientation of the customer's teeth is adjusted such that the outer surfaces of the customer's teeth of the upper jaws and/or lower jaws forms a substantially continuous curve and/or the gaps between the teeth are harmonized. The target state may be defined individually according to aesthetic views. The application software may automatically compute and suggest at least one target state depending on a catalogue of images of aesthetically pleasant teeth.
   ∘ Sub-Step A6-2: Computing at least one intermediate position and/or orientation of at least two teeth or of all teeth of the upper jaws and/or lower jaws of the customer representing an intermediate state during dental treatment with the at least one aligner, preferably based on the at least one digital image. This Sub-Step aims to correct and/or visualize the alignment of the customer's teeth during the dental self-treatment using the aligners. An intermediate state may be a state after the customer's teeth have been aligned with a dental aligner except for the last aligner to be used in terms of the dental self-treatment. Same as above, this Sub-Step may help to advertise and promote the services offered by the claimed method but it is even more important for the design of the dental aligners to be used in context with the dental self-treatment in order to accomplish teeth alignment in accordance with the computed intermediate position and/or orientation of the customer's teeth.
   ∘ Step A6-3: Generating at least one simulated view of the least two teeth or of all teeth of the upper jaws and/or lower jaws of the customer during or after completion of the dental self-treatment based on the computation in Sub-Step A6-1 and/or Sub-Step A6-2, preferably on the basis of the at least on digital image, more preferably by modifying the at least on digital image based on the computation in Sub-Step A6-1 and/or Sub-Step A6-2. This Sub-Step may enable the customer to more precisely assess the results and progress of the dental self-treatment over the time. Such information may affect the customer's decision to order a specific treatment option out of several treatment options. As an example, the customer may see from the simulated views that the dental self-treatment may provide better results with a higher number of different dental aligners as compared to a lower number of different dental aligners. In such case, the customer may be encouraged to opt for a more expensive treatment program involving a higher number of dental aligners, such that the visualization of simulated treatment results becomes an important aspect in terms of marketing.
   ∘ Sub-Step A6-4: Providing the at least one simulated view to the customer, preferably in electronic form. The simulated view can be provided to the customer via the application software.
   ∘ Sub-Step A6-5: Developing or modifying a treatment plan based on the computation in Sub-Step A6-1 and/or Sub-Step A6-2. The computed final and/or intermediate state(s) may of course be used for visualization purposes but also for adaptation of the treatment plan with a view to administering the most efficient treatment to the customer.
   ∘ Sub-Step A6-6: Providing the treatment plan to the customer, preferably in electronic form. In order to involve the customer as far as possible into the dental self-treatment and make the treatment transparent, the treatment plan can be available to the customer, preferably via the application software, as accomplished in this Sub-Step.
- Sub-Step A7: Assessing a progress of the dental self-treatment based on at least one in-treatment or post-treatment digital image of the customer's teeth made during or after the dental self-treatment, preferably including at least one of the following Sub-Steps:
   ∘ Sub-Step A7-1: Rendering a message to the customer, inviting the customer to (regularly) provide at least one in-treatment or post-treatment digital image of the customer's teeth to be made during or after the dental treatment, preferably by upload. Sub-Step A7 initiates a target-performance comparison, wherein the actual treatment results are compared with the intended or predicted treatment results. Such target-performance comparison requires additional input from the customer in terms of monitoring, accompanying and documenting the progress of the dental self-treatment with digital images.
   ∘ Sub-Step A7-2: Executing Sub-Step A4 and/or Sub-Step A5 on the at least one in-treatment or post-treatment digital image of the customer's teeth. In addition to the at least one pre-treatment digital image that was taken before the dental self-treatment, the in-treatment or post-treatment digital images can be rendered subject to image processing according to Sub-Step A4 and/or Sub-Step A5 in order to evaluate of the physical condition of the customer's teeth during the dental self-treatment in an automated procedure. By parameterizing the in-treatment or post-treatment digital images and comparing the parameter values attributed to digital images with preset parameter requirements, it is possible to automatically verify whether or not the dental self-treatment is successful. Insofar, the claimed method still does not require the interaction of dentists and dental laboratories for monitoring the progress of the dental self-treatment. Only in case it is determined that the progress of the dental self-treatment is not successful, dentists and dental laboratories may be involved. Hence, this Sub-Step assists the automated monitoring of the progress of the dental self-treatment and may automatically interrupt the dental self-treatment if it is determined that the dental self-treatment is not successful or if any complications occur.
   ∘ Sub-Step A7-3: Setting a parameter requirement for at least one treatment progress parameter representative of an acceptable treatment progress, said at least one treatment progress parameter preferably including the treatment-related change of position and/or orientation of at least one tooth or of at least two teeth or of all teeth of the upper jaws and/or lower jaws of the customer, said treatment progress parameter requirement being more preferably editable and/or derived from a catalogue of images related to customers with successful treatments. Same as in preceding steps, this Sub-Step quantifies a treatment progress. As the dental self-treatment aims at the alignment of teeth, the current customer's teeth alignment status - expressed in terms of position and/or orientation of the customer's teeth - is taken as a reference for the treatment progress assessment.
   ∘ Sub-Step A7-4: Parameterizing the at least one in-treatment or post-treatment digital image so as to determine at least one treatment progress parameter value from the at least one in-treatment or post-treatment digital image, and, if applicable, the at least one pre-treatment digital image of the customer's teeth made before the dental self-treatment, for enabling comparison with the treatment progress parameter requirement set in Sub-Step A7-3, preferably using object recognition technology, more preferably by comparing the at least one in-treatment or post-treatment digital image of the customer's teeth with at least one pre-treatment digital image of the customer's teeth, and by computing a discrepancy level between said images. This step allows quantifying the treatment progress in terms of parameter values attributed to the treatment related change of position and/or orientation of the customer's teeth.
   ∘ Sub-Step A7-5: Verifying whether or the at least one treatment progress parameter value determined in Sub-Step A7-4 complies with the parameter requirement set in Sub-Step A7-3. In other words, this Sub-Step aims to determine on the basis of parameter values derived from the digital images of the customer's teeth whether or not the dental self-treatment is successful up to an intermediate state or the final state of the treatment.
   ∘ Sub-Step A7-6: If the verification in Sub-Step A7-5 is negative, adjusting a treatment plan and/or a computation of a target state, and preferably rendering a message to the customer, informing the customer on the unsuccessful treatment progress. As such, the determination of unsuccessful dental self-treatment enables the possibility of intervention and modification of the treatment plan, as accomplished in this Sub-Step.
   ∘ Sub-Step A7-7: If the verification in Sub-Step A7-5 is positive, proceeding with the method according to the present treatment plan, and preferably repeating Sub-Step A7 until the dental self-treatment according to the treatment plan has been completed, and more preferably rendering a message to the customer, informing the customer on the successful treatment progress. This Sub-Step assists the customer as well as the service provider to confirm that the dental self-treatment proceeds as expected. This Sub-Step further secures that only customers with successful intermediate treatment results are administered the full treatment, so that resources will be used most efficiently.
- Sub-Step A8: Verifying whether or not the at least one digital image is available in or convertible into a digital file format for 3D-printing, preferably an STL-file format, more preferably including at least one of the following Sub-Steps:
   ∘ Sub-Step A8-1: If the verification in Step A8 is negative, rendering a message to the customer inviting the customer to upload at least another digital image of the customer's teeth that is available in or convertible into a digital file format for 3D-printing and/or triggering shipment of a 3D-imaging kit to the customer, preferably on demand. This step secures that the process continues even though the available image does not satisfy the requirements for the creation of 3D-printing files.
   ∘ Sub-Step A8-2: Verifying whether or not the at least another digital image provided by the customer relates to the same customer, and if the verification is negative, repeating Sub-Step A8-2 with any other digital image until the verification is positive. This routine is repeated until a digital image is available that allows the process to continue.
   ∘ Sub-Step A8-3: Executing Sub-Step A4 on the at least another digital image, and preferably proceeding with Step B if the verification in Sub-Step A4 is positive. Image eligibility requirements of course should be checked for any digital image to be processed in context with creating the 3D-printing file, which is accomplished in this Sub- Step.

In the easiest case, the digital image that is used for image eligibility assessment and customer eligibility assessment directly enables the creation of a 3D-printing file, even though this is not necessary. It is also possible that image eligibility assessment and customer eligibility assessment are performed on the basis of at least one 2D-image, for example a photography taken by a camera of the customer's smartphone. The procurement of such 2D-digital image for image eligibility assessment and customer eligibility assessment may be easier and cheaper as compared to the procurement of a 3D-digital image for the creating of the 3D-printing file in view of the hardware resources required therefore. As a single 2D-image lacks the information of the third dimension, the creation of a 3D-printing file is not possible from a single 2D-image. However, if different 2D-digital images of the same target object are available from different viewpoints, modern image processing software allows calculating the information of the third dimension from the two images such that the creation of the 3D-printing file would be possible from two or more 2D-images. Modern smartphones with dual lens or triple lens technology allow taking of multiple images of a target object from different viewpoints, enabling 3D-images and the creation of 3D-printing files from such set of images directly. Insofar, the same digital image that is used for image and customer eligibility assessment may also be used for the creation of 3D-printing files. If this is not possible, the customer will be invited to provide at least another digital image that allows the creation of 3D-printing files. As the customer itself may not be in the possession of suitable hardware equipment for taking a digital image that allows the creation of 3D-printing files, such hardware equipment may be made available to the customer on a preliminary basis for this specific purpose.

It can prove beneficial if Step B comprises at least one of the following Sub-Steps:
- Sub-Step B1: Creating at least one 3D-printing file based on at least one 3D-image of the customer's teeth and/or based on at least two 2D-images of the customer's teeth taken from different view angles. Therefore, different sources of digital images, i.e. both 3D-as well as 2D-images, could be used for creating the 3D-printing file, which opens the process for different imaging technologies.
- Sub-Step B2: Creating at least one 3D-printing file based on the target state computed in Sub-Step A6-1 and/or the intermediate state computed in Sub-Step A6-2. As such, dental aligners can be produced not only for a target state but also for one, two or even more intermediate states, depending on the magnitude of dental alignment required by the customer.
- Sub-Step B3: Storing the at least one 3D-printing file on a data storage device, preferably on the same data storage device from which the at least one digital image has been loaded in Sub-Step A2. Therefore, the dental aligners can be printed with any 3D-printer anywhere and anytime.

It also could prove helpful if the method further comprises Step C: 3D-printing the at least one dental aligner according to the at least one 3D-printing file created in Step B. The step of printing the dental aligner is a preferred but nonessential step of the claimed method. Once the 3D-printing file is available, the 3D-printing can be carried out on any private or public 3D-printer. This printing step is therefore not necessarily part of a commercial service offered by the service provider.

An independent aspect of the claimed invention relates to a data processing system comprising means for carrying out the steps of the method according to one of the preceding embodiments. The claimed method can be implemented with the data processing system. The data processing system may involve private and/or public components.

It can prove helpful if the data processing system comprises at least one of the following items:
- At least one processor, which preferably comprises at least one of the following features:
   ∘ The at least one processor is configured to load a program comprising instructions to perform at least a part of the method according to one of the claims 1 to 4, preferably Step A and/or Step B and/or Step C or any of its/their Sub-Steps.
   ∘ The at least one processor forms part of a computer, preferably a server computer or a smartphone.
- At least one data storage device, which preferably comprises at least one of the following features:
   ∘ The at least one data storage device is integrally combined with the at least one processor. This feature allows simple data transfer between both items. It is, however, not essential that the data storage device is integrally combined with a processor, as the data storage device may be provided separately or remotely from the processor and linked via (remote) communication means.
   ∘ The at least one data storage device forms part of a computer, preferably a server computer or a smartphone.
   ∘ The at least one data storage device is configured to store the program comprising instructions to perform at least a part of the method according to one of the claims 1 to 4, preferably Step A and/or Step B and/or Step C or any of its/their Sub-Steps.
   ∘ The at least one data storage device is configured to store the at least one digital image of the customer's teeth.
   ∘ The at least one data storage device is linked with the processor, preferably via LAN or the internet.
   ∘ The at least one data storage device is configured to store a plurality of digital images of customer's teeth in customer accounts in combination with a customer ID allotted to each digital image. This feature proves helpful in order to implement the claimed invention on a commercial scale.
   ∘ The at least one data storage device is configured to permit the customer to access the customer's account upon authentication. With this feature, the customer can actively use the data storage device as a private filebox e.g. for uploading digital images and/or downloading treatment plans provided on the data storage device.
- At least one data input/output device, which preferably comprises at least one of the following features:
   ∘ The least one data input/output device is integrally combined with the at least one processor and/or the at least one data storage device. This allows easy and secure data handling as well as data transfer between the components of the data processing system.
   ∘ The least one data input/output device is configured to permanently or at least preliminarily communicate with the at least one processor and/or the at least one data storage device, preferably via LAN or via the internet.
   ∘ The at least one data input/output device forms part of a computer, preferably a server computer or a smartphone.
   ∘ The least one data input/output device allows to input and/or output data to be used and/or data used in the method according to one of the claims 1 to 4.
   ∘ The least one data input/output device is configured to output acoustic and/or visual signals related to the method according to one of the claims 1 to 4.
   ∘ The least one data input/output device is configured to capture acoustic and/or visual and/or haptic commands and convert to input data related to the method according to one of the claims 1 to 4.
   ∘ The least one data input/output device comprises a display, preferably a touch-display, and/or speakers.
   ∘ The least one data input/output device comprises acoustic and/or visual input means configured to capture acoustic and/or visual input signals.

The data input/output device according to at least one of the above features enhances communication between the service provider and the customer, so that the customer can be actively involved in the process towards the creation of the 3D-printing file for the dental aligner that is finally used for dental self-treatment of the customer.
- At least one digital imaging device, which preferably comprises at least one of the following features:
   ∘ The least one digital imaging device is integrally combined with the at least one processor and/or the at least one data storage device and/or the at least one data input/output device. This allows easy and secure data handling as well as data transfer between the components of the data processing system.
   ∘ The least one digital imaging device is configured to permanently or at least preliminarily communicate with the at least one processor and/or the at least one data storage device and/or the at least one data input/output device, preferably via LAN or via the internet.
   ∘ The at least one digital imaging device forms part of a computer, preferably a server computer or a smartphone.
   ∘ The at least one digital imaging device is configured to take the at least one digital image of the customer's teeth.
   ∘ The at least one digital imaging device is configured to take a 2D-image and/or a 3D-image of the customer's teeth, the image preferably being a photo or scan.
   ∘ The at least one digital imaging device is or comprises a 2D-camera.
   ∘ The at least one digital imaging device is or comprises a 3D-camera.
   ∘ The at least one digital imaging device or at least one of its functions is controllable via the processor and/or the data input/output device, wherein the at least one controllable function preferably includes: image format, image focus, image size, image resolution, sharpness, brightness, contrast, exposure time.
   ∘ The at least one digital imaging device or at least one of its functions is controllable by the movement of the imaging device.
   ∘ The at least one digital imaging device is embodied as handheld device, preferably a camera stick.
   ∘ The at least one digital imaging device comprises an image sensor and is controllable by the image sensor, so that the imaging device automatically starts taking images once the image sensor assumes the presence of teeth in the image to be taken.
   ∘ The at least one digital imaging device is configured to transfer the at least digital image onto the data storage device, preferably wireless, more preferably via Bluetooth or NFC.
   ∘ The at least one digital imaging device is configured to take intraoral images of a customer's upper jaws and/or lower jaws (see e.g. Fig. 3).
   ∘ The at least one digital imaging device comprises a digital imaging device having a grip for extraorally holding the digital imaging device and an image sensor for intraorally imaging the customer's upper jaws and/or lower jaws, wherein preferably the digital imaging device has an elongate body wherein the grip and the image sensor are arranged at opposite ends of the elongate body (see e.g. Fig. 3).

If the data processing system includes a one digital imaging device according to at least one of the above features, the compatibility with the other hardware components of the data processing system can be secured.
- Preferably a 3D-printer, configured to 3D-print the at least one dental aligner for dental self-treatment of a customer according to the 3D-printing file created on the basis of at least one digital image of the customer's teeth. For the reasons given above, the 3D-printer is an optional hardware component of the data processing system as the 3D-printer might also be privately owned and not part of the commercially viable system providing the services according to the present invention.

Another independent aspect of the present invention relates to a computer program comprising instructions preferably causing the system according to one of the claims 5 or 6 to execute the steps of the method according to one of the claims 1 to 4, wherein the computer program preferably comprises at least one of the following instructions:
- Instruction 11: Controlling the data input/output device so as to input/output data assisting the customer in handling the at least one digital image device for taking the at least one digital image. This instruction usually follows a procedure of registration of the customers contact, shipping and payment data. The guidance information provided to the customer may include acoustic and/or visual information using speakers and/or a display, respectively. Such information may include a video tutorial informing the customer how to adjust the camera settings for taking the initial digital images to be subjected to the image and customer eligibility checks. The video tutorial may be opened by the customer by pressing on a button appearing on a display screen. The video tutorial may instruct the customer to adjust the camera settings according to the image quality requirements. It is also possible that the customer confers certain rights to the application software installed on his own device to automatically access and adjust settings in other application software, such as a camera function. In such case, the application software can be programmed user-friendly so that the user is only required to take a minimum of actions.
- Instruction 12: Controlling at least one function of the at least one digital image device while taking the at least one digital image. This instruction proves helpful and user-friendly, in particular if the customer has conferred respective rights to the application software installed on his own device.
- Instruction 13: Controlling the processor so as to transfer the at least one digital image from the at least one digital image device onto the at least one data storage device. Again, this instruction proves helpful and user-friendly, in particular if the customer has conferred respective rights to the application software installed on his own device.
- Instruction 14: Controlling the data input/output device so as to input/output data assisting the customer in performing the dental self-treatment using the at least one dental aligner. The information provided to the customer may include acoustic and/or visual information using speakers and/or a display, respectively. Such information may include a video tutorial informing the customer how and when and how long to wear the dental aligner, how to clean the dental aligner, and how to monitor the progress of the self-treatment, and the like.

It may prove advantageous if instruction 11 comprises at least one of the following commands:
- Command 11-1: Controlling the data input/output device so as to output acoustic and/or visual instructions for instructing the customer to start operating and/or moving of the at least one digital imaging device.
- Command 11-2: Controlling the data input/output device so as to output acoustic and/or visual instructions for instructing the customer to move the at least one digital imaging device into a specific position for taking at least one of an outside view image and/or an inside view image of the customer's upper jaws and/or lower jaws, a bottom view image of customer's upper jaws and/or a top view image of the customer's lower jaws.

- Command 11-3: Controlling the data input/output device so as to output acoustic and/or visual instructions for instructing the customer to move the digital imaging device on a specific path along the inside and/or the outside of the customer's upper jaws and/or lower jaws from one end to the other end thereof.
- Command 11-4: Controlling the data input/output device so as to output acoustic and/or visual instructions for instructing the customer to change path and/or speed and/or direction of movement of the at least one digital imaging device.
- Command 11-5: Controlling the data input/output device so as to output acoustic and/or visual instructions for instructing the customer to move the digital imaging device to other portions of the upper jaws and/or the lower jaws of the customer than the portions which have been recognized from the at least one digital image, and repeating this step until the customer's upper jaws and/or lower jaws have been completely recognized from the at least one digital image (preferably upon verification in Sub-Step A4-3 that at least one image content parameter value determined in Sub-Step A4-2 is not within the parameter requirement set in Sub-Step A4-1).
- Command 11-6: Controlling the data input/output device so as to output acoustic and/or visual instructions for instructing the customer to stop moving and/or operating the at least one digital imaging device, preferably upon recognition that the customer's teeth present in the upper jaws and/or lower jaws are completely depicted in the at least one digital image.

The above identified commands may help the customer to efficiently provide suitable digital images that meet the preset image eligibility requirements to be checked by the program.

It may further prove beneficial if instruction 12 comprises at least one of the following commands:
- Command 12-1: Controlling the at least one digital imaging device to (automatically) start imaging (upon receipt of an image starting command).
- Command 12-2: Controlling the at least one digital imaging device to (automatically) stop imaging (upon receipt of an image stopping command).
- Command 12-3: Controlling the at least one digital imaging device to (automatically) store the at least one digital image (upon receipt of an image storing command).
- Command 12-4: Controlling the at least one digital imaging device to (automatically) adjust at least one function, preferably the format and/or size and/or resolution and/or brightness and/or contrast and/or sharpness of the at least one digital image, more preferably upon the verification in Sub-Step A4-3 that the at least one image quality parameter value determined in Sub-Step A4-2 is not within the parameter requirement set in Sub-Step A4-1.

The above identified commands are directed to the digital imaging device and contribute to make the services particularly user-friendly.

Another preferred aspect of the present invention relates to a handheld digital imaging device, said digital imaging device being configured to be operated by a customer for taking at least one intraoral digital 3D-image of the customer's upper jaws and/or lower jaws and to transfer the at least one intraoral digital 3D-image onto a data storage device, wherein preferably the at least one digital imaging device comprises at least one of the features according to claim 6.d. The digital imaging device can be a camera with a 3D-imaging function.

Still another preferred aspect of the present invention relates to a starter kit for assisting a customer in taking at least one digital image of the customer's teeth in context with the method according to any of the claims 1 to 4, the starter kit preferably comprising at least one of the following items:
- At least one cheek holder.
- At least one mirror.
- Usage instructions, instructing the customer how to take the at least one digital image using the at least one cheek holder and the at least one mirror, and instructing the customer how to initiate Step A of the method according to any of the claims 1 to 4.
- At least one link allowing the customer access the data processing system according to any of the claims 5 or 6, or to download the computer program method according to any of the claims 7 to 9, preferably an URL or QR code (e.g. for App download, customer data, instructions etc).

Yet another preferred aspect of the present invention relates to an imaging kit for assisting a customer in taking at least one digital image of the customer's teeth that allows creating at least one 3D-printing file in context with Step B of the method according to any of the claims 1 to 4, the imaging kit preferably comprising at least one of the following items:
- At least one cheek holder.
- At least one mirror.
- At least one digital imaging device for taking at least one digital image of the customer's teeth that can be used for creating a 3D-printing file in Step B of the method according to any of the claims 1 to 4, wherein preferably the at least one digital imaging device comprises at least one of the features according to claim 6.d.
- Unique identifier for activation of at least one digital imaging device.
- Usage instructions, instructing the customer how to take the at least one digital image using the at least one cheek holder, the at least one mirror and at least one digital imaging device, and instructing the customer how to initiate Step B of the method according to any of the claims 1 to 4.

At least one link allowing the customer access the data processing system according to any of the claims 5 or 6 (e.g. for App download, customer data, instructions etc).

Yet another preferred aspect of the present invention relates to an aligner kit for allowing and assisting the customer in performing the dental self-treatment, the Aligner kit preferably comprising at least one of the following items:
- At least one dental aligner that has been 3D-printed on the basis of the 3D-printing file created in accordance with the computer-implemented method according to one of the claims 1 to 4.
- At least one cheek holder
- At least one mirror
- Usage instructions, instructing the customer how to use the at least one dental aligner.
- QR codes (e.g. for App download, customer data, instructions etc)
- At least one treatment plan, preferably developed according to Sub-Step A7-4, more preferably in written or digital form, even more preferably including scheduled checkpoints (which customer approves).
- FAQs including what to do if problems or questions should occur.

Further preferred embodiments result from combinations of the features disclosed in the claims, description and drawings.

### Terms and Definitions

### Digital image

The term "digital image" includes any digital representation of a target object. Preferred image types are photographs and preferred file formats are JPG or BMP. However, there is no limitation with respect to a specific type of image or file format. The claimed method is computer-implemented. Hence, the "digital image" may be available in any computer-readable form. The "digital image" does not have to be a visible image, i.e. it does not require a visual representation of the target object may just correspond to a set of digital coordinates.

More specifically, the term "digital image of the customer's teeth" includes any digital representation of the customer's teeth that enables the image and/or customer eligibility assessment and/or the creation of a 3D-printing file of a dental aligner fitting to the customer's teeth for performing the dental self-treatment. For this purpose, the digital image of the customer's teeth can be any digital representation that contains at least the information of the shape and/or position and/or orientation of at least two teeth but preferably of all teeth of the upper jaws and/or lower jaws of the customer. The dental aligner basically corresponds to the negative form of outer surface and/or inner surface and/or chewing surface of at least two teeth but preferably of all teeth of the upper jaws and/or lower jaws of the customer, wherein the position and/or orientation of at least one tooth, preferably the relative position and/or orientation of at least two teeth, even more preferably the relative position and/or orientation of all teeth of the upper jaws and/or lower jaws of the customer is modified compared to the pre-treatment state. The minimum resolution of the digital image and/or the 3D-printing file preferably corresponds to the resolution of the 3D-printer used for 3D-printing the dental aligner, such that there is precise information available for each pixel to be printed. Hence, the digital image of the customer's teeth may be an image based on vector graphics and the like.

The "digital image of the customer's teeth" may be reduced to the essential information. Information of the teeth surroundings, i.e. shape and condition of the gum or oral mucosa may be dispensable and does not have to be included in the digital image.

The at least one digital image preferably satisfies at least one of the following requirements:
- The at least one digital image is or comprises at least one digital representation.
- The at least one digital image is or comprises at least one 2D-image and/or at least one 3D-image.
- The at least one digital image of the customer's teeth includes information on the shape and/or position and/or orientation of at least some teeth of the upper jaws and/or lower jaws of the customer, such information being preferably given in terms of coordinates.

### 3D-printing file

The preferred digital file format for the 3D-printing file is STL.

STL (an abbreviation of "stereolithography") is a file format native to the stereolithography CAD software created by 3D-Systems. STL has several after-the-fact backronyms such as "Standard Triangle Language" and "Standard Tessellation Language". This file format is supported by many other software packages; it is widely used for rapid prototyping, 3D-printing and computer-aided manufacturing. STL files describe only the surface geometry of a three-dimensional object without any representation of color, texture or other common CAD model attributes. The STL format specifies both ASCII and binary representations. Binary files are more common, since they are more compact.

An STL file describes a raw, unstructured triangulated surface by the unit normal and vertices (ordered by the right-hand rule) of the triangles using a three-dimensional Cartesian coordinate system. In the original specification, all STL coordinates were required to be positive numbers, but this restriction is no longer enforced and negative coordinates are commonly encountered in STL files today. STL files contain no scale information, and the units are arbitrary.

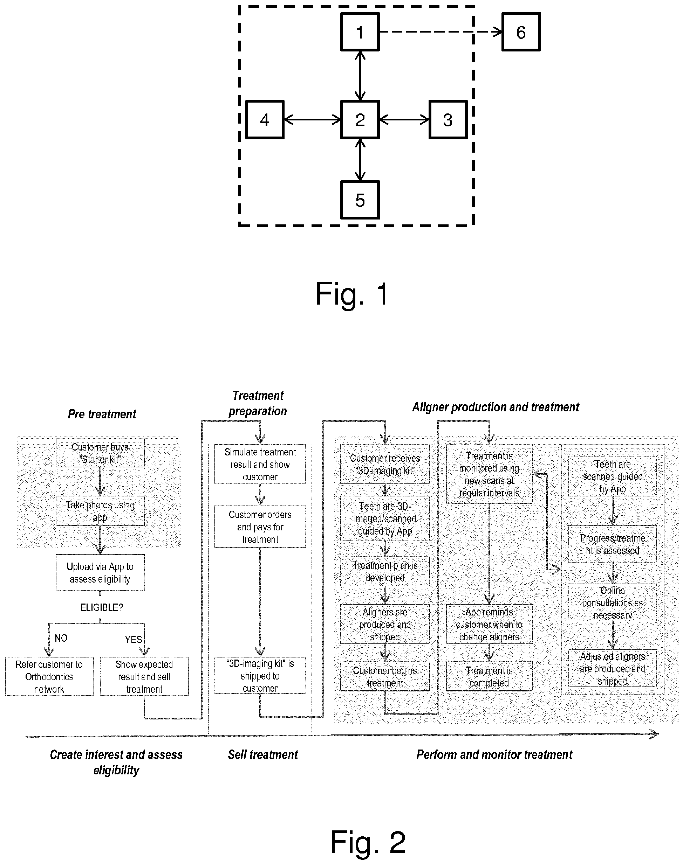

The basic principle is to take a regular 2D-image (preferably in BMP/JPEG format) and translate the image's gray shades into a 3D-height map (Figures 1 and 2). The two-dimensional array (i.e. color) of each element is interpreted as a height value in order to store information on the height of each point. For example, the white points are flat, the gray points are higher and the black points are the highest.

Of course, it is also possible other digital file formats for the 3D-printing file.

### Computer

The term computer is not limited to a specific piece of hardware. It may be any electronic item that comprises a processor and is capable of performing at least some of the steps of the computer-implemented method according to the invention. Exemplary items covered by the term computer are PCs, smartphones, tablet computers, PDAs, server-computers and the like.

### Digital requirement

A digital requirement is a requirement that can be set and verified by a computer routine or algorithm by mathematic operations based on digital values.

One of such digital requirement corresponds to the customer's eligibility for dental self-treatment. That is, the customer is only eligible for dental self-treatment if the misalignment of the customer's teeth does not exceed specific values.

### Application software

In context with the present invention, the term application software designates a computer program to be installed on a public (i.e. publicly accessible) or private computer, preferably on the customer's private smartphone, which, when loaded, performs at least one Step or Sub-Step of the computer-implemented method according to claim 1 to 4.

### Service Provider

In context with the present invention, the service provider is a natural or legal person that offers commercial services through the computer-implemented method according to claim 1 to 4, preferably through the application software.

### Brief description of drawings

Fig. 1 is a schematic diagram illustrating the components of the data processing system and their interactions in context with the computer-implemented method according to the present invention.
Fig. 2 is high level process map of the computer-implemented method according to the present invention.
Fig. 3 schematically shows how the imaging device is to be used for taking the digital image of the customer's teeth.
Fig. 4 schematically shows how individual digital images depicted in views (a) and (b) can be combined to form one combined image depicted in view (c).
Fig. 5 shows schematic views of exemplary digital images of customer's teeth subjected to the image eligibility assessment according to Step A of the claimed method, wherein view (a) depicts an exemplary digital image that fails the image quality assessment due to insufficient brightness (e.g. the detected brightness parameter value 0,5 does not comply the preset brightness parameter requirement from 0,8 to 1,2) and view (b) depicts an exemplary digital image that fails the image content assessment as it does not cover all teeth of the customer's upper jaws (e.g. the image shows only the teeth 14 to 17 according to the teeth scheme completely, so that the image content parameter value attributed to the digital image does not comply the preset image content requirement of teeth 11 to 17 and 21 to 27 according to the teeth scheme).
Fig. 6 shows schematic views of exemplary digital images of customer's teeth subjected to the customer eligibility assessment according to Step A of the claimed method, wherein views (a) and (b) depict exemplary digital images of customer's upper and lower jaws, respectively, that have passed the image quality assessment, wherein views (c) and (d) schematically depict boundary lines in the digital images according to views (a) and (b) which indicate that the customer is eligible for the dental self-treatment with dental aligners as all of the customer's teeth are within the maximum parameter requirements specified in terms of shape, position and orientation of teeth.

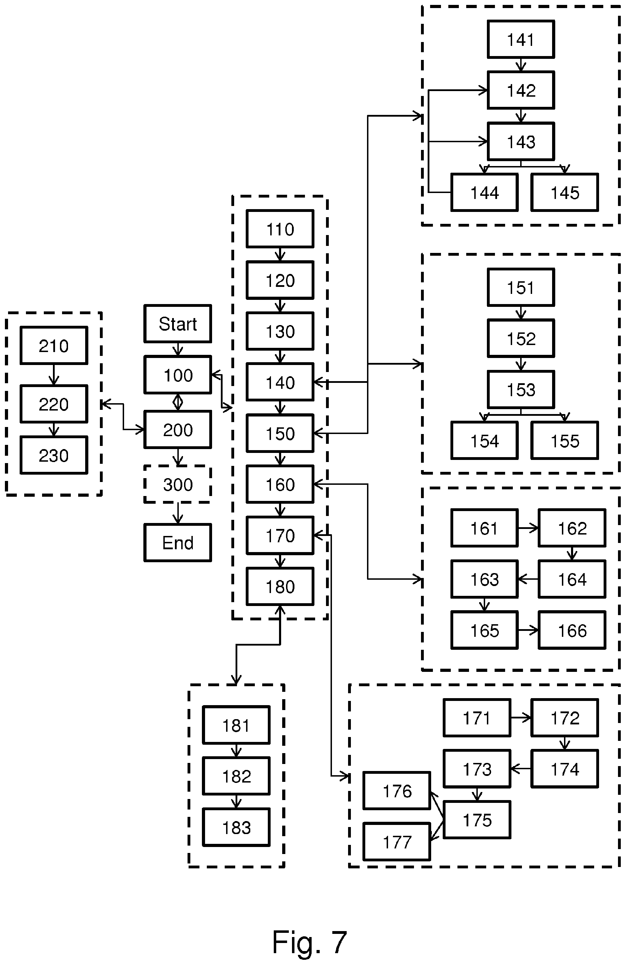

Fig. 7 is a flowchart illustrating the Steps and Sub-Steps of the computer-implemented method according to the present invention.

### Detailed description of preferred embodiments

The present invention provides a unique dental service to its customers. It provides beautiful smiles via minor teeth correction(s) using unique digital technologies in a personal interaction with its customers and collaboration with dental laboratories.

Created and designed by highly experienced dental professionals (Orthodontist, Implantologist, leading Dental laboratories) the dental treatment is very accurate, high quality, fast and the cost is far less than that of conventional alternatives.

Dental aligners are a proven successful dental device for many years and used by millions of customers/patients. The present invention provides a new approach for preparing such dental aligners, namely by digital interaction and personalization all the way to delivering the aligners to the customers without the need of a physical visit at a dental or other office, which is unique.

Hence, the present invention provides a fully digital dental service specialized in providing aligner treatments with reassurance that the best of Orthodontics dentists and dental laboratories are taking care of the customer and the customer's teeth.

### Embodiment 1

In the first embodiment, the data processing system comprises the following components/items for carrying out the steps of the computer-implemented method according to the present invention: server computer 1, smartphone 2, data-storage device 3, smartphone camera 4, 3D-camera (pen scanner) 5, 3D-printer 6. Fig. 1 schematically depicts the components of the data processing system as boxes in Fig. 1, wherein the arrows between the boxes schematically represent the data exchange between these components.

The centerpiece of customer interaction is via the application software (App) to be installed and executed on the customer's smartphone 2. The customer's smartphone 2 is to be connected to the service provider server's server computer 1, preferably via the Internet. The application software contains algorithms for assessing image and/or customer eligibility (Sub-Step A4 and/or Sub-Step A5), simulating treatment plans (Sub-Step A6 and/or Sub-Step A7), conducting 3D-scans via a 3D-camera 5, designing customer treatments, preparing and monitoring treatment plans and conducting video-consultations between customers and the service provider, Orthodontics specialists and support staff. The customer is an integrated part of the process and works real-time together with the technology in producing the foundation for the treatment and the aligner(s) themselves.

### Process step overview:

Fig. 2 is high level process map of the computer-implemented method according to the first embodiment.

### Physical components

The following physical components are involved in the computer-implemented method according to the first embodiment.

The "Starter kit" contains equipment to be used by the customer for initiating the computer-implemented method according to the first embodiment and taking the digital images to be used in context with the method. The "Starter kit" is to be shipped to the customer in a box and includes inter alia the following key components:
- Cheek holder(s)
- Mirror(s)
- Usage instructions
- QR codes (e.g. for App download, customer data, instructions etc)

The "3D-imaging kit" contains equipment to be used by the customer for taking the at least one digital image that serves as a basis for the creation of the 3D-printing file; it is to be shipped to the customer in a box and includes inter alia the following key components:
- Cheek holder(s) and mirror(s)
- A 3D-imaging device
- Usage instructions
- QR codes (e.g. for App download, customer data, instructions etc)
- Unique identifier of Device (for activation)

The "Aligner" box contains equipment to be used by the customer for performing the dental self-treatment using the dental aligner that has been 3D-printed according to the 3D-printing file created in accordance with the computer-implemented method according to the first embodiment. The "Aligner" box is to be shipped to the customer after 3D-printing at least the first dental aligner in any form that secures safe transport of the dental aligner and includes inter alia the following key components:
- Aligner for treatment
- Cheek holder(s) and mirror(s)
- Usage instructions
- QR codes (e.g. for App download, customer data, instructions etc)
- Written treatment plan including checkpoints scheduled (which customer approves)
- FAQs including what to do if problems or questions should occur

### Entry point - start of process

The entry point for the customer to start the process can be a website, application software to be installed on device private computer or via an URL (e.g. QR code) on the "Starter kit" box or "3D-imaging kit" box.

The customer can order and pay for the "Starter kit" from the website, via the application software or through third parties who retail the "Starter kit".

### Taking and uploading the digital images (photos)

The application software and/or the website provide access to instructional videos/animations or other illustrations on how to take the photos using the equipment in the "Starter kit" (Sub-Step A1).

Via the application software, the customer launches the photography function of the application software, which takes the photos of the customer's teeth, both upper and lower jaws, as well as taking photos using the mirrors and the cheek holders in the "Starter kit". The application software may provide an interface such as a graphical user interface (GUI) and/or speech control interface for the user. The interface may enable user to launch photography function. The application software establishes connection between the customer's smartphone 2 and the server computer 1 and uploads the photos to data-storage device 3 allocated to the server computer 1 (Sub-Step A2). Separate digital images showing parts of the customer's teeth may be combined to form a combined image that allow a full assessment in terms of image and customer eligibility Sub-Step A3. A routine to be executed on the server computer 1 upon completion of the upload of the photos monitors the completeness and quality of the photos taken by the customer (Sub-Step A4). The application software may require access to certain functions of the customer's smartphone 2 in order to store the photos taken by the customer on a data-storage device 3 of the customer's smartphone 2 (Step Sub-A2), e.g. during preliminary disconnection with the server computer 1. The application software interacts with the customer on progress and quality of the photos (Sub-Step A4), guides them through the photo process and when photos are sufficient for customer eligibility assessment, the software on the server computer 1 assesses the customer's photos of the teeth using object recognition technology and assesses against a catalogue of eligible teeth (Sub-Step A5). The interaction may be provided by displaying in the smartphone display.

If the verification results in that the customer eligibility requirements have been satisfied, the application software communicates this to the customer via the user interface and the customer can proceed on the application software to simulate what results of the treatment could be (Sub-Steps A6 and A7). The result of simulation may be displayed on the display of the smartphone.

If the verification results in that the customer eligibility requirements have not been satisfied, i.e. the customer's teeth are not eligible for dental treatment with dental aligners, the application software can direct the customer to Orthodontics Dentists who can help them. This may be performed by displaying on the display of the smartphone contact information or web page of the Dentist. The application may provide the customer/user with the an interface element (such as a button or a link), which - when activated by a customer/user - controls the smartphone to connect with the Dentist, for example by automatically dialing the corresponding telephone number or opening a chat or generating an email or the like.

### Simulation of the treatment results (Steps A6 and A7)

The application software provides the customer with the option to see a simulation of what the result of a treatment would look like. If the customer chooses this, the simulation software on the server computer 1 is activated. Using the actual photos of the customer's teeth, the software uses treatment algorithm to simulate what the results of a treatment could be and the application software displays this to the customer.

The application software displays it to the customer on the display of the customer's smartphone 2 and asks the customer to confirm that he wants to go ahead with a dental treatment.

The application software can also show the customer what the next steps in a treatment would be in an educational video (i.e. starting with a 3D-scan of the mouth using the 3D-scanning kit, construction of a treatment plan, construction of production specifications, production of aligners and shipment, regular monitoring of progress of treatment and potentially video-consultations until the treatment is completed.

The customer can choose to order the full treatment immediately or order the 3D-scanning kit for the 3D-scanning.

### Customer orders treatment

Via the application software or the website, the customer can order the treatment or order the 3D-imaging kit.

If the customer orders the 3D- imaging kit only, the customer pays for the 3D-imaging kit and it is shipped to the customer.

If the customer orders the dental treatment, the customer pays for the entire treatment (which includes the shipment of the 3D-imaging kit). It is possible to order a base treatment, which includes several checkpoints at which the customer will re-scan his/her teeth and progress will be assessed, or include video-consultation or other services in the treatment.

The customer is offered financing at the time of ordering (i.e. payments split over a time period).

### Customer scans teeth using the 3D-imaging device

Both the website and the application software contain detailed instructional videos on how to use the 3D-imaging kit for 3D-imaging the customer's teeth.

3D-imaging device 5 is linked via Bluetooth to the customer's smartphone 2 and preferably real-time linked to the server computer 1 containing the scanning software (Instruction 11).

Following the instructions, the customer scans the entire mouth - upper and lower jaws - and the application software is constantly monitoring the scanning and ensuring that both the completeness and accuracy are of sufficient quality (Instruction 12). The 3D-scan, which is a 3D-digital representation of the customer's teeth, preferably in an STL-format, allows a final assessment that treatment is possible and enables the software to construct a treatment plan by the software and finally to convert this treatment plan into production specifications according to which the aligners will be produced.

The 3D-imaging process is an interaction between the server computer 1 and the customer via the application software, which controls the customer's smartphone 2 to upload the 3D-scans and receives analysis (Instruction 13), and where the application software guides the customer on where and how to scan and ending up with a completed scan. When this is done, the application software communicates to the customer that the scan is complete.

Depending on the 3D-image/scan, i.e. the digital representation of the customer's teeth, which includes information on the current/initial shape, position and orientation of the customer's teeth, the software calculates one or more improves alignments of the customer's teeth, so that e.g. gaps between neighboring teeth will be eliminated and/or the outer surfaces of the neighboring teeth are substantially flush with each other, and set each improved alignment as potential target state. The software may further calculate how many different aligners will be required in order to get from the initial/current state to the respective target state as each aligner may only cause a limited change of position and/or orientation of the customer's teeth. The software may further calculate one or more intermediate states corresponding to the position and orientation of the customer's teeth accomplished by the use of a specific aligner. Depending on the number of required dental aligners, the software may calculate a treatment plan to be forwarded to the customer along with the dental aligners.

The next steps depend on whether the customer has already purchased the treatment plan or has only purchased the 3D-imaging kit.

If the customer has only purchased the 3D-imaging kit, the customer has now to decide whether to order the dental treatment or not. The application software can control the display 3 of the customer's smartphone 2 to show what the result after the dental treatment will look like (Sub-Step A6). The simulation is more accurate than before if it is based on a real 3D-scan. If the customer wants to proceed with the treatment, payment for the treatment has to be made. The customer has to sign electronically and accept that he/she wants a dental treatment, as required by some national laws. The aligners can now be produced and sent to the customer.

If the customer has already purchased the dental treatment, the application software now communicates to the customer what the dental treatment will be using the treatment plan. The treatment plan has been created based on the 3D-scan (Sub-Step A6-6). The dental aligners can now be produced and sent to the customer.

The customer gets a written confirmation and overview of the treatment plan calculated by the software, e.g. by displaying on the display of the smartphone.

### Dental treatment

When the Aligner Box has arrived at the customer, the treatment begins. Detailed instructional videos are available on the website and how to use them and are also available on the application software or via URL (e.g. QR code) on the Aligner Box (Instruction 14).

When the customer starts the treatment, he/she notifies this in the application software in order for the application software to keep track of upcoming aligner changes and progress check points.

During the course of the treatment, the application software will remind the customer to change the aligners.

Progress of the dental treatment is monitored for the entirety of the treatment for all customers (Sub-Step A7). At certain predetermined points in the treatment (as determined when the treatment plan was designed) and depending on what treatment the customer has chosen initially, a progress check is conducted again using a new scan with the 3D-scanning kit by the customer.

By having the customer conduct a re-scan of their teeth using the 3D-scanning kit together with the application software, the software will compare the new position of the teeth with the projected position according to the treatment plan (Sub-Step A7-5).

If the treatment is on track, the application software will inform the customer of that real-time (Sub-Step A7-7).

If the treatment is not on track, the application software, preferably automatically or in consultation with Orthodontics experts, will recalculate the treatment plan and communicate what needs to be done to the customer (Sub-Step A7-6). This could include inter alia a) changing the sequence of the aligners the customer already has received (e.g. going back one step), b) producing new aligners for the customer or c) scheduling a (video)- consultation with Orthodontics experts to discuss the situation with the customer.

The steps above are repeated multiple times during the process until the treatment is completed.

### Treatment completed

When the treatment is completed, the application software informs the customer that the treatment is now complete. The application software asks the customer to take one last post-treatment photo and uploads it.

The customer may do a testimonial via the application software's testimonial functionality which can then be used for promotional purposes.

The application software offers the customer a feature where they can easily share (before and after) photos etc. on Facebook, Instagram etc.

### Reference signs list

- 1: Server computer
- 2: Customer's private computer / Smartphone
- 3: Data storage device
- 4: Data input/output device
- 5: 3D-imaging device/ 3D- camera stick
- 5a: 3d-image sensor / imaging head / lens
- 5b: grip
- 6: 3D-printer

## Claims

1. Computer-implemented method for creating at least one 3D-printing file of at least one dental aligner for dental self-treatment of a customer based on at least one digital image of the customer's teeth, wherein the method includes the following steps:
a. Step A (100): Verifying whether or not the at least one digital image satisfies preset requirements on image eligibility and/or customer eligibility.
b. Step B (200): If the verification in Step A is positive, creating the at least one 3D-printing file for 3D-printing the at least one dental aligner based on the at least one digital image.

2. Computer-implemented method according to claim 1, **characterized in that** Step A comprises at least one of the following Sub-Steps:
a. Sub-Step A1 (110): Triggering shipment of a starter kit to the customer for assisting the customer in taking the at least one digital image.
b. Sub-step A2 (120): Loading the at least one digital image, preferably from a data storage device (2) onto which the at least one digital image has been uploaded (e.g. by the customer).
c. Sub-step A3 (130): Combining at least two different of said digital images to form a combined digital image of the customer's teeth, wherein the combined digital image preferably depicts at least some or all teeth of the upper jaws and/or lower jaws of the customer, wherein more preferably said combined digital image replaces the at least one digital image in at least one of the subsequent method steps.
d. Sub-Step A4 (140): Verifying whether or not the at least one digital image satisfies preset image eligibility requirements on image quality and/or image content, preferably including at least one of the following Sub-Steps:
i. Sub-Step A4-1 (141): Setting a parameter requirement for at least one image eligibility parameter representative of eligible image quality and/or eligible image content, said at least one image eligibility parameter preferably including the format and/or size and/or resolution and/or brightness and/or contrast and/or sharpness of the at least one digital image, and/or the number and/or type of teeth depicted in at least one digital image, said image eligibility parameter requirement being more preferably editable and/or derived from a catalogue of eligible images.
ii. Sub-Step A4-2 (142): Parameterizing the at least one digital image so as to determine at least one parameter value from the at least one digital image for enabling comparison with the parameter requirement set in Sub-Step A4-1 (141).
iii. Sub-Step A4-3 (143): Verifying whether or not the at least one parameter value determined in Sub-Step A4-2 (142) complies with the parameter requirement set in Sub-Step A4-1 (141).
iv. Sub-Step A4-4 (144): If the verification in Sub-Step A4-3 is negative, rendering a message to the customer, inviting the customer to provide at least another digital image in order to satisfy preset image eligibility requirements, and/or repeating Sub-Step A2 and/or Sub-Step A3 (143) on the basis of the at least another digital image.
v. Sub-Step A4-5 (145): If the verification in Step A4-3 is positive, proceeding with the method, preferably with Sub-Step A5 (150).
e. Sub-Step A5 (150): Verifying whether or not the at least one digital image satisfies preset customer eligibility requirements, preferably including at least one of the following Sub-Steps:
i. Sub-Step A5-1 (151): Setting a parameter requirement for at least one customer eligibility parameter representative of an eligible customer for dental self-treatment with the at least one dental aligner, said at least one customer eligibility parameter preferably including the shape and/or size and/or position and/or orientation of at least one tooth of the customer, more preferably the relative shape and/or size and/or position and/or orientation of at least at least two teeth of an eligible customer, wherein the at least two teeth are even more preferably two adjacent teeth or all (present) teeth of the upper jaws and/or lower jaws of an eligible customer, said customer eligibility parameter requirement being even more preferably editable and/or derived from a catalogue of images of eligible customers.
ii. Sub-Step A5-2 (152): Parameterizing the at least one digital image so as to determine at least one parameter value from the at least one digital image for enabling comparison with the parameter requirement set in Sub-Step A5-1 (151), preferably using object recognition technology.
iii. Sub-Step A5-3 (153): Verifying whether or the at least one parameter value determined in Sub-Step A5-2 (152) complies with the parameter requirement set in Sub-Step A5-1 (151).
iv. Sub-Step A5-4 (154): If the verification in Step A4 is negative, rendering a message to the customer, informing the customer on its ineligibility for the dental self-treatment with dental aligners, and/or referring the customer to a dentist.
v. Sub-Step A5-5 (155): If the verification in Sub-Step A5-4 is positive, proceeding with the method, preferably with Sub-Step A6, more preferably by rendering a message to the customer, informing the customer on its eligibility for the dental self-treatment with dental aligners and/or running a routine for showing the customer what the results of a treatment would look like and/or running a routine for performing a payment procedure and/or running a routine for triggering shipment of a 3D-imaging kit to the customer.
f. Sub-Step A6 (160): Simulating the effects of the dental self-treatment of the customer, preferably based on the at least one digital image of the customer's teeth, more preferably including at least one of the following Sub-Steps:
i. Sub-Step A6-1 (161): Computing a target position and/or orientation of at least two teeth or of all teeth of the upper jaws and/or lower jaws of the customer representing a target state upon completion of dental self-treatment with the at least one aligner (1), preferably based on the at least one digital image.
ii. Sub-Step A6-2 (162): Computing at least one intermediate position and/or orientation of at least two teeth or of all teeth of the upper jaws and/or lower jaws of the customer representing an intermediate state during dental treatment with the at least one aligner, preferably based on the at least one digital image.
iii. Sub-Step A6-3 (163): Generating at least one simulated view of the least two teeth or of all teeth of the upper jaws and/or lower jaws of the customer during or after completion of the dental self-treatment based on the computation in Sub-Step A6-1 and/or Sub-Step A6-2, preferably on the basis of the at least on digital image, more preferably by modifying the at least on digital image based on the computation in Sub-Step A6-1 and/or Sub-Step A6-2.
iv. Sub-Step A6-4 (164): Providing the at least one simulated view to the customer, preferably in electronic form.
v. Sub-Step A6-5 (165): Developing or modifying a treatment plan based on the computation in Sub-Step A6-1 and/or Sub-Step A6-2.
vi. Sub-Step A6-6 (166): Providing the treatment plan to the customer, preferably in electronic form.
g. Sub-Step A7 (170): Assessing a progress of the dental self-treatment based on at least one in-treatment or post-treatment digital image of the customer's teeth made during or after the dental self-treatment, preferably including at least one of the following Sub-Steps:
i. Sub-Step A7-1 (171): Rendering a message to the customer, inviting the customer to (regularly) provide at least one in-treatment or post-treatment digital image of the customer's teeth to be made during or after the dental treatment, preferably by upload.
ii. Sub-Step A7-2 (172): Executing Sub-Step A4 (140) and/or Sub-Step A5 (150) on the at least one in-treatment or post-treatment digital image of the customer's teeth.
iii. Sub-Step A7-3 (173): Setting a parameter requirement for at least one treatment progress parameter representative of an acceptable treatment progress, said at least one treatment progress parameter preferably including the treatment-related change of position and/or orientation of at least one tooth or of at least two teeth or of all teeth of the upper jaws and/or lower jaws of the customer, said treatment progress parameter requirement being more preferably editable and/or derived from a catalogue of images related to customers with successful treatments.
iv. Sub-Step A7-4 (174): Parameterizing the at least one in-treatment or post-treatment digital image so as to determine at least one treatment progress parameter value from the at least one in-treatment or post-treatment digital image, and, if applicable, the at least one pre-treatment digital image of the customer's teeth made before the dental self-treatment, for enabling comparison with the treatment progress parameter requirement set in Sub-Step A7-3 (174), preferably using object recognition technology, more preferably by comparing the at least one in-treatment or post-treatment digital image of the customer's teeth with at least one pre-treatment digital image of the customer's teeth, and by computing a discrepancy level between said images.
v. Sub-Step A7-5 (175): Verifying whether or the at least one treatment progress parameter value determined in Sub-Step A7-4 (174) complies with the parameter requirement set in Sub-Step A7-3 (173).
vi. Sub-Step A7-6 (176): If the verification in Sub-Step A7-5 is negative, adjusting a treatment plan and/or a computation of a target state, and preferably rendering a message to the customer, informing the customer on the unsuccessful treatment progress.
vii. Sub-Step A7-7 (177): If the verification in Sub-Step A7-5 is positive, proceeding with the method according to the present treatment plan, and preferably repeating Sub-Step A7 until the dental self-treatment according to the treatment plan has been completed, and more preferably rendering a message to the customer, informing the customer on the successful treatment progress.
h. Sub-Step A8 (180): Verifying whether or not the at least one digital image is available in or convertible into a digital file format for 3D-printing, preferably an STL-file format.
i. Sub-Step A8-1 (181): If the verification in Step A8 is negative, rendering a message to the customer inviting the customer to upload at least another digital image of the customer's teeth that is available in or convertible into a digital file format for 3D-printing and/or triggering shipment of a 3D-imaging kit to the customer, preferably on demand.
ii. Sub-Step A8-2 (182): Verifying whether or not the at least another digital image provided by the customer relates to the same customer, and if the verification is negative, repeating Sub-Step A8-2 (182) with any other digital image until the verification is positive.
iii. Sub-Step A8-3 (183): Executing Sub-Step A4 (140) on the at least another digital image, and preferably proceeding with Step B if the verification in Sub-Step A4 (140) is positive.

3. Computer-implemented method according to one of the preceding claims, **characterized in that** Step B comprises at least one of the following sub-steps:
a. Sub-Step B1 (210): Creating at least one 3D-printing file based on at least one 3D-image of the customer's teeth and/or based on at least two 2D-images of the customer's teeth taken from different view angles.
b. Sub-Step B2 (220): Creating at least one 3D-printing file based on the target state computed in Sub-Step A6-1 (161) and/or the intermediate state computed in Sub-Step A6-2 (162).
c. Sub-Step B3 (230): Storing the at least one 3D-printing file on a data storage device (2), preferably on the same data storage device (2) from which the at least one digital image has been loaded in Sub-Step A2 (120).

4. Computer-implemented method according to one of the preceding claims, wherein the method further comprises Step C (300): 3D-printing the at least one dental aligner according to the at least one 3D-printing file created in Step B.

5. Data processing system comprising means for carrying out the steps of the method according to one of the preceding claims.

6. Data processing system according to claim 5, comprising the following items:
a. At least one processor, which preferably comprises at least one of the following features:
i. The at least one processor is configured to load a program comprising instructions to perform at least a part of the method according to one of the claims 1 to 4, preferably Step A and/or Step B and/or Step C or any of its/their Sub-Steps.
ii. The at least one processor forms part of a computer, preferably a server computer (1) or a smartphone (2).
b. At least one data storage device (3), which preferably comprises at least one of the following features:
i. The at least one data storage device (3) is integrally combined with the at least one processor (1).
ii. The at least one data storage device (3) forms part of a computer, preferably a server computer (1) or a smartphone (2).
iii. The at least one data storage device (3) is configured to store the program comprising instructions to perform at least a part of the method according to one of the claims 1 to 4, preferably Step A and/or Step B and/or Step C or any of its/their Sub-Steps.
iv. The at least one data storage device (3) is configured to store the at least one digital image of the customer's teeth.
v. The at least one data storage device (3) is linked with the processor, preferably via LAN or the internet.
vi. The at least one data storage device (3) is configured to store a plurality of digital images of customer's teeth in customer accounts in combination with a customer ID allotted to each digital image.
vii. The at least one data storage device (3) is configured to permit the customer to access the customer's account upon authentication.
c. At least one data input/output device (4), which preferably comprises at least one of the following features:
i. The least one data input/output device (4) is integrally combined with the at least one processor and/or the at least one data storage device (3).
ii. The least one data input/output device (4) is configured to permanently or at least preliminarily communicate with the at least one processor and/or the at least one data storage device (4), preferably via LAN or via the internet.
iii. The at least one data input/output device (4) forms part of a computer, preferably a server computer or a smartphone.
iv. The least one data input/output device (4) allows to input and/or output data to be used and/or data used in the method according to one of the claims 1 to 4.
v. The least one data input/output device (4) is configured to output acoustic and/or visual signals related to the method according to one of the claims 1 to 4.
vi. The least one data input/output device (4) is configured to capture acoustic and/or visual and/or haptic commands and convert to input data related to the method according to one of the claims 1 to 4.
vii. The least one data input/output device (4) comprises a display, preferably a touch-display, and/or speakers.
viii. The least one data input/output device (4) comprises acoustic and/or visual input means configured to capture acoustic and/or visual input signals.
d. At least one digital imaging device (5), which preferably comprises at least one of the following features:
i. The least one digital imaging device (5) is integrally combined with the at least one processor and/or the at least one data storage device (3) and/or the at least one data input/output device (4).
ii. The least one digital imaging device (5) is configured to permanently or at least preliminarily communicate with the at least one processor and/or the at least one data storage device (3) and/or the at least one data input/output device (4), preferably via LAN or via the internet.
iii. The at least one digital imaging device (5) forms part of a computer, preferably a server computer or a smartphone.
iv. The at least one digital imaging device (5) is configured to take the at least one digital image of the customer's teeth.
v. The at least one digital imaging device (5) is configured to take a 2D-image and/or a 3D-image of the customer's teeth, the image preferably being a photo or scan.
vi. The at least one digital imaging device (5) is or comprises a 2D-camera.
vii. The at least one digital imaging device (5) is or comprises a 3D-camera.
viii. The at least one digital imaging device (5) or at least one of its functions is controllable via the processor and/or the data input/output device (4), wherein the at least one controllable function preferably includes: image format, image focus, image size, image resolution, sharpness, brightness, contrast, exposure time.
ix. The at least one digital imaging device (5) or at least one of its functions is controllable by the movement of the imaging device (5).
x. The at least one digital imaging device (5) is embodied as handheld device, preferably a camera stick.
xi. The at least one digital imaging device (5) comprises an image sensor and is controllable by the image sensor, so that the imaging device (5) automatically starts taking images once the image sensor assumes the presence of teeth in the image to be taken.
xii. The at least one digital imaging device (5) is configured to transfer the at least digital image onto the data storage device (3), preferably wireless, more preferably via Bluetooth or NFC.
xiii. The at least one digital imaging device (5) is configured to take intraoral images of a customer's upper jaws and/or lower jaws.
xiv. The at least one digital imaging device (5) comprises a digital imaging device (5) having a grip (5b) for extraorally holding the digital imaging device (5) and an image sensor (5a) for intraorally imaging the customer's upper jaws and/or lower jaws, wherein preferably the digital imaging device (5) has an elongate body wherein the grip and the image sensor are arranged at opposite ends of the elongate body.
e. Preferably a 3D-printer (6), configured to 3D-print the at least one dental aligner for dental self-treatment of a customer according to the 3D-printing file created on the basis of at least one digital image of the customer's teeth.

7. Computer program comprising instructions to cause the system according to one of the claims 5 or 6 to execute the steps of the method according to one of the claims 1 to 4, wherein the computer program preferably comprises at least one of the following instructions:
a. Instruction 11: Controlling the data input/output device (4) so as to input/output data assisting the customer in handling the at least one digital image device (5) for taking the at least one digital image.
b. Instruction 12: Controlling at least one function of the at least one digital image device (5) while taking the at least one digital image.
c. Instruction 13: Controlling the processor so as to transfer the at least one digital image from the at least one digital image device (5) onto the at least one data storage device (3).
d. Instruction 14: Controlling the data input/output device (4) so as to input/output data assisting the customer in performing the dental self-treatment using the at least one dental aligner.

8. Computer program method according to claim 7, wherein instruction 11 comprises at least one of the following commands:
a. Command 11-1: Controlling the data input/output device (4) so as to output acoustic and/or visual instructions for instructing the customer to start operating and/or moving of the at least one digital imaging device (5).
b. Command 11-2: Controlling the data input/output device (4) so as to output acoustic and/or visual instructions for instructing the customer to move the at least one digital imaging device (5) into a specific position for taking at least one of an outside view image and/or an inside view image of the customer's upper jaws and/or lower jaws, a bottom view image of customer's upper jaws and/or a top view image of the customer's lower jaws.
c. Command 11-3: Controlling the data input/output device (4) so as to output acoustic and/or visual instructions for instructing the customer to move the digital imaging device on a specific path along the inside and/or the outside of the customer's upper jaws and/or lower jaws from one end to the other end thereof.
d. Command 11-4: Controlling the data input/output device (4) so as to output acoustic and/or visual instructions for instructing the customer to change path and/or speed and/or direction of movement of the at least one digital imaging device (5).
e. Command 11-5: Controlling the data input/output device (4) so as to output acoustic and/or visual instructions for instructing the customer to move the digital imaging device (5) to other portions of the upper jaws and/or the lower jaws of the customer than the portions which have been recognized from the at least one digital image, and repeating this step until the customer's upper jaws and/or lower jaws have been completely recognized from the at least one digital image, preferably upon verification in Sub-Step A4-3 (143) at least one image content parameter value determined in Sub-Step A4-2 (142) is not within the parameter requirement set in Sub-Step A4-1 (141).
f. Command 11-6: Controlling the data input/output device (4) so as to output acoustic and/or visual instructions for instructing the customer to stop moving and/or operating the at least one digital imaging device (5), preferably upon recognition that the customer's teeth present in the upper jaws and/or lower jaws are completely depicted in the at least one digital image.

9. Computer program according to claim 7 or 8, wherein instruction 12 comprises at least one of the following commands:
a. Command 12-1: Controlling the at least one digital imaging device (5) to (automatically) start imaging (upon receipt of an image starting command).
b. Command 12-2: Controlling the at least one digital imaging device (5) to (automatically) stop imaging (upon receipt of an image stopping command).
c. Command 12-3: Controlling the at least one digital imaging device (5) to (automatically) store the at least one digital image (upon receipt of an image storing command).
d. Command 12-4: Controlling the at least one digital imaging device (5) to (automatically) adjust at least one function, preferably the format and/or size and/or resolution and/or brightness and/or contrast and/or sharpness of the at least one digital image, more preferably upon the verification in Sub-Step A4-3 (143) that the at least one image quality parameter value determined in Sub-Step A4-2 (142) is not within the parameter requirement set in Sub-Step A4-1 (141).

10. Handheld digital imaging device, said digital imaging device (5) being configured to be operated by a customer for taking at least one intraoral digital 3D-image of the customer's upper jaws and/or lower jaws and to transfer the at least one intraoral digital 3D-image onto a data storage device (3), wherein preferably the at least one digital imaging device (5) comprises at least one of the features according to claim 6.d.

11. Starter kit for assisting a customer in taking at least one digital image of the customer's teeth in context with the method according to any of the claims 1 to 4, the starter kit preferably comprising at least one of the following items:
a. At least one cheek holder
b. At least one mirror
c. Usage instructions, instructing the customer how to take the at least one digital image using the at least one cheek holder and the at least one mirror, and instructing the customer how to initiate Step A of the method according to any of the claims 1 to 4.
d. At least one link allowing the customer access the data processing system according to any of the claims 5 or 6, or to download the computer program method according to any of the claims 7 to 9, preferably an URL or QR code (e.g. for App download, customer data, instructions etc).

12. Imaging kit for assisting a customer in taking at least one digital image of the customer's teeth that allows creating at least one 3D-printing file in context with Step B of the method according to any of the claims 1 to 4, the Imaging kit preferably comprising at least one of the following items:
a. At least one cheek holder
b. At least one mirror
c. At least one digital imaging device (5) for taking at least one digital image of the customer's teeth that can be used for creating a 3D-printing file in Step B of the method according to any of the claims 1 to 4, wherein preferably the at least one digital imaging device (5) comprises at least one of the features according to claim 6.d.
d. Unique identifier for activation of at least one digital imaging device (5).
e. Usage instructions, instructing the customer how to take the at least one digital image using the at least one cheek holder, the at least one mirror and at least one digital imaging device (5), and instructing the customer how to initiate Step B of the method according to any of the claims 1 to 4.
f. At least one link allowing the customer access the data processing system according to any of the claims 5 or 6 (e.g. for App download, customer data, instructions etc).

13. Aligner kit for allowing and assisting the customer in performing the dental self-treatment, the Aligner kit preferably comprising at least one of the following items:
a. At least one dental aligner that has been 3D-printed on the basis of the 3D-printing file created in accordance with the computer-implemented method according to one of the claims 1 to 4.
b. At least one cheek holder
c. At least one mirror
d. Usage instructions, instructing the customer how to use the at least one dental aligner.
e. QR codes (e.g. for App download, customer data, instructions etc)
f. At least one treatment plan, preferably developed according to Sub-Step A7-4, more preferably in written or digital form, even more preferably including scheduled checkpoints (which customer approves).
g. FAQs including what to do if problems or questions should occur.
